# EUROPEAN PATENT SPECIFICATION

(11) **EP 1 389 064 B1**
(45) Date of publication and mention of the grant of the patent: **04.08.2010**
(21) Application number: 02715702.3
(22) Date of filing: 22.01.2002
(51) Int. Cl.: A61B 8/00, A61H 5/00, A61M 37/00

(54) **APPARATUS FOR THE DELIVERY OF SUBSTANCES TO BIOLOGICAL COMPONENTS**
APPARAT ZUR ABGABE VON SUBSTANZEN AN BIOLOGISCHE KOMPONENTEN
APPAREIL PERMETTANT LA DELIVRANCE DE SUBSTANCES A DES COMPOSANTS BIOLOGIQUES

(30) Priority: 26.01.2001 IL 14112301
(43) Date of publication of application: 18.02.2004
(73) Proprietor: Iger, Yoni, 34601 Haifa (IL)
(72) Inventor: Iger, Yoni, 34601 Haifa (IL)
(74) Representative: Moore, Barry
(86) International application number: PCT/IL2002/000056
(87) International publication number: WO 2002/058530

(56) References cited:
- EP-A1- 0 246 515
- EP-A1- 0 258 637
- EP-A2- 0 642 802
- WO-A-01/00094
- WO-A-98/18391
- WO-A-98/32379
- WO-A-98/48711
- DE-A- 2 032 433
- DE-A1- 3 324 575
- US-A- 5 315 998
- US-A- 5 618 275
- US-A- 5 656 016
- US-A- 6 018 678
- US-A- 6 135 976

## Description

### FIELD OF THE INVENTION

The present invention concerns a device for the delivery of accelerated substances, soluble or particulate, to and through biological components such as membranes, organelles, cells, tissues, organs or creatures, using ultrasound as a preferred accelerating agent and while isolating the biological component from the driven force.

### BACKGROUND OF THE INVENTION

Needle less delivery of substances such as mechanical stabilizers, drugs, nutrients, gene-carriers, vaccines or metabolites, either as particles or in solution, into natural or artificial biological components, is often faced with difficulties due to mat-penetration attributed to the barriers functioning against undesired penetration of foreign components. Also topical delivery to internal zones of biological components is faced with difficulties associated with mal-permeability of biological component.

Ionphoresis, high pressure injection, or ultrasound are among the techniques developed for the facilitation of efficient and safe administration of substances into biological components, mostly of superficial zones.

For example, ultrasound is used for facilitation of transport of various compounds across tissues, typically skin (Mitragotri, M., et al., Science, 269:850-853 (1995)).

The ultrasonic delivery was improved by using ultrasound in conjunction with chemical permeation enhancer and/or iontophoresis (U.S. Patent 5,231,975). Other methods use ultrasonic waves to excite the local nerves, thereby to open the epidermal/dermal junction membrane and the capillary endothelial cell joints, which enables the transfer of drugs through the skin and into the blood stream (U.S. Patent No. 5,421,816) or delivery through two pulses where the first one enlarges the intercellular spaces and the second one enables delivery thereof (PCT/IL97/00405). Significant problem of the conventional ultrasound, ionphoresis or chemical assisted delivery is that during the process the biological component is constantly exposed to the driven stimulus, such as irradiation.

It is also desirable to deliver into biological components relatively large amounts of solutions, or complex particles. State-of-the-art ultrasound-facilitated administration methods are unsuitable for administration of said solutions or complex particles, since application of ultrasound pulses, sufficient to drive a small amount of small-sized molecule through a tissue is insufficient to drive large amounts or to drive those complex particles through tissues or biological or artificial membranes. Increase of the duration, or intensity, changes of frequency or of the ultrasound pulses to levels which are presumably sufficient to drive the large amounts of solutions or particles through the tissue or cell membrane in one operation, or a serial of repeated operations, has not been reported probably since it results in irreversible damage to the tissue and in significant cell-death. Similarly, irreversible damage occurs in non-biological membranes of e.g., polyethylene or elastomer (for example those used in implants), when increased intensities or durations of ultrasound irradiation have been used.

Other devices perform delivery of compounds by employing a pressure enforced from compressed gas reservoir or by gas spring to create sufficient pressure enabling pushing of medication through e.g., the skin tissue (U.S. Patent No 6,096,002). Significant problems here include the need of high-pressure gas reservoir, moving pistons, or gas release, which restricts application to only certain external tissues.

At times, it is desirable to deliver into biological components substances in the form of solutions, or particles, without accompanied energy delivery to the biological component, without gasses flux or moving pistons. It is also desired to do substances administration regardless of their molecular weight, ionic condition, size or polarity.

It would have been highly desirable to provide a method for a single as well as high repeatability delivery of wide variety of substances to natural or artificial biological components, either superficial or internal, utilizing driving force, while isolating the driving force from the biological components, therefore minimizing the damage to the tissue or cells. It would have further been desirable to provide an ultrasound facilitated method for delivery of solutions or complex particles having a relatively large size and without employing pressure to the compound to be delivered, nor ventilation or energy delivery to the treated area.. It is the object of this invention to provide a device for multi purposes intra tissual delivery, which avoid limitations of current technologies and reduce their possible side effects.

Examples of the known art include the disclosures of WO9848711 US6135976, WO9818391, DE3324575, WO0100094, WO9832379.

US patent no. 5,618,275 discloses the features of the preamble of claim 1. The document shows low frequency ultrasonic pressure waves of high intensity which are applied to the skin to cause cavitation. A therapeutic agent may be applied to the skin in a predetermined amount prior to the application of the pressure waves. The therapeutic agent may also be applied to the skin subsequent to the application of the pressure waves. Ultrasonic pressure waves of high frequency which do not cause cavitation may be applied to the skin after delivery of a therapeutic agent. The depth of penetration of the agent may be controlled by varying the burst width of electrical signals applied to a piezoelectric transducer. A housing surrounds the transducer and a sleeve may be provided to define with the housing a predetermined controlled volume chamber for holding a measured amount of agent. An ultrasonic power supply includes means for generating both low and high ultrasonic frequency electric signals, and a switch alternatively connects the high and low frequency signals to the transducer.

### SUMMARY OF THE INVENTION

Accordingly the invention provides a device according to claim 1 with advantageous embodiments provided in the dependent claims.

In view of the above, the present invention provides a novel method and device allowing the delivery of substances to, into or through biological components that are part of or an entire biological entity. Said biological components might be membranes, organelles, cells, tissues, organs or creatures. This, in accordance with the invention, is achieved by utilizing an ultrasound stimulus, or other energy source capable of producing high acceleration rate over short distance and at high repeatability, to accelerate the substance to be delivered via low density medium and in the direction of the biological component. By accelerating the substance attached to an ultrasonic vibrating element, or other high accelerating means, in a low density medium, it has been found that substances continue to move in direction of acceleration and it was possible to deliver substances while affecting only the attached substance and isolating the biological component from the energy source, therefore enabling substance delivery without energy delivery to the biological component and without causing it energy related damage. The delivery of substances to biological compounds shall be preferably performed via low density medium such as gas or vacuum. The delivery does not involve any gas streaming or moving parts, and is applicable also to internal tissues.

The use of the device according to the invention comprises the step of exposing the substance to be delivered to a high amplitude ultrasound stimulus, being such as to accelerate the ultrasound attached substance, kept at certain distance from the biological component, via low density medium and in the direction of the said biological component. Surface of the ultrasound generating element, might be covered by compounds capable of reducing the surface-tension, therefore enabling easy release of substance to be delivered. Due to the distance filled with low density medium between the energy source and the substance to be delivered on one hand, and the biological component on the other hand, said energy created by the accelerating means is markedly attenuated in the low density medium and essentially do not reach the target biological component. On the other hand, the substance to be delivered is accelerated with minimal friction during delivery and eventually at least part of it reaches and penetrate the biological component. The disconnection between the accelerating agent, for instance the ultrasound, and the biological component, as well as the non pressurized procedure, enable delivery without causing damage to the bulk of said biological component, at high repetition rate and also to internal zones of biological components, as is below explained..

The device of the present invention may be used for therapeutic and cosmetic purposes, as well as for diagnostic and experimental purposes, according to the type of substance to be delivered, and the relevant biological component.

By one non limiting embodiment, the substances to be administered may be soluble substances such as various medicaments for therapeutic treatment, anti ageing agents for prevention purposes, toxic compounds for controlled degeneration, growth factors hormones or interleukins for the initiation or cessation of processes, amino acids or proteins or substrate elements to be resources for macromolecules or processes, macromolecules such as DNA molecules or their fragments, for the purpose of gene therapy or genetic manipulation, various dyes for the purpose of diagnosis inside cells, or within a tissue, substances for local anesthesia, substances for topical destruction of biological component, substances for the reduction or acceleration the activity of biological component or of any sub biological component including infectious agents, substances for changing the mechanical or chemical properties of a biological component or any of it's subunits, and the like.

By yet another non-limiting embodiment, the substances to be administered are complex particles. The term *"particles"* or *"complex particle"* refers generally to a particle having the size of at least 1 nm ranging to tens or hundreds of microns which is usually composed of a single type of molecule, or alternatively several types of molecules. The complex particles are essentially insoluble in the medium in which they are carried. Examples of complex particles are granules of toxic compounds, sensitizers or radioactive compounds, attenuated or killed disease-causing agents or parts thereof such as bacteria, virions, fungi, protozoa or parasites administered for the purpose of vaccination; plasmids containing DNA to be inserted for the purpose of gene-therapy or genetic manipulations; nano-particles with genes or DNA vaccines, nanomachines, nuclei of gametes administered into oocytes for the purpose of fertilization; particles impregnated with medicaments capable of releasing them at a slow rate to the surrounding tissue for the purpose of therapy or controlled immune reduction; particles containing compounds that were coated with a protective coating, for example, in order to form particles having different solubility, to prevent oxidation, to prevent a hygroscopic effect, to increase resistance to heat or to protect the contents of the particle from biological effects (such as degradation); particles comprising a biologically compatible dye for the purpose of tattooing, as for example, in the case of permanent makeup; particles comprising a detectable marker for the purpose of diagnosis, and the like.

According to another non limiting example, particles might be also inert or other compounds of particular characteristics, accelerated towards biological components at high acceleration rates, from short distance and at certain angle, and affecting by disconnecting, causing destruction and removal of sub-components or layers of said treated biological components.

Particles or solutions might be also active or inert compounds used for mechanical support or stabilization of biological components. Active or inert particles might be also used for paving of biological components.

The *"biological component"* to which the substances are administered, can be any type of membranes, organelles, cells, tissues, organs or creatures. Biological component might therefore refer to eukaryotic or prokaryotic cells, or their sub-components, including cells cultured in a medium. Biological component might further refer to epithelial tissues which may be keratinized epithelial tissues such as skin, or moist- epithelial tissues, for example, the epithelium lining the eyes, digestive tract, respiratory, or reproductive systems. The tissue may also be the moist epithelial tissue covering aquatic creatures such as fish, crustaceans or mollusks at different stages of rearing, including embryonic ones.

The term *"biological component"* might also refer to artificial components, being parts of, or replacing parts of, or assisting the activity of, or used as mechanical support for, or used for releasing substances to or through natural membranes, organelles, cells, tissues, organs or creatures. Therefore the term biological component might refer also to elastomer compounds which form part of an implant, or to artificial skin which has been constructed for replacing damaged skin area, to encapsulated cells or artificial tissue constructed for slow release, or similar artificial components, as the case might be.

The substance accelerating stimuli, created by ultrasound or any other accelerating mean, are applied when the relevant biological component is not in contact with the accelerating stimuli mean, nor in contact with any liquid medium or gel coupling medium that form a bridge between the biological component and the accelerating mean, but remains isolated from the accelerating stimuli when that is being performed. The medium between the stimulating element and the biological component is essentially composed of an ultrasound isolation medium, such as gas or vacuum, and not of ultrasound coupling medium.

The substance to be administered shall be acoustically coupled to the stimulating element at least during part of the operation period. That is to say that coupling might be on permanent or temporal basis. Temporal coupling might be achieved for instance, during at least part of cycle of the acceleration of the vibrating element towards the substance. The substance may be present in liquid, gel, paste, powder, pellet, solid strip and the like. It might be composed of homogenous materials or alternatively of different compounds mixed together close to the vibrating element before the delivery, or mixed in the space between vibrating element and biological component during delivery, or mixed in the biological component after the delivery.

According to non-limiting embodiment, more then one compound is delivered to gain the desired effect. This according to the invention can be performed by having same accelerating rates to the different compounds, or alternatively performing different accelerating rates due to substance weight or size, or by delivery from more than one vibrating element and more than one substance-supply sub-devices. When more than one stimulus is being given, the stimuli may be applied one after the other or simultaneously.

The specific parameters of the stimulus, capable of driving the administered substances into or through said biological components, should be determined empirically, depending among other things on the nature of the biological component, on the nature of the administered substance and on the parameters of the accelerating mean. However, at least one stimuli composed of at least cycle portion shall be given to deliver unit of substance.
Generally speaking, the driving stimulus has the following parameters: Frequency: At least 1 Hz; Preferably 10 Hz to 30 MHz, more preferably 10 kHz to 3 MHz, most preferably, 20 kHz to 100 kHz. Duration: At least quarter of cycle; Therefore at least 0.025 sec. or 0.75x10⁻⁷ sec for 10 Hz or 30 MHz respectively. Amplitude: At least one micron; Preferably 10 to 10,000 microns, most preferably 20 to 200 microns. Intensity: 0.0001 - 10,000 W/cm², preferably 0.1 - 100 W/cm², most preferably 3-50 W/cm². Under preferred embodiment, the ultrasonic force is used to cause acceleration of substance to be delivered to the site of administration in the biological component.

It shall be understood that also in the ultrasonic range of frequencies, certainly below it, also other means might be used to create the acceleration force. Such means might include any means that can produce high acceleration rates, over short distance of movement and at high repeatability, for instance sonic speakers, electromagnets, motors, motor-coupled ex-centers, liquid-containing pistons and the like.

Generally speaking, the acceleration rate can be determined as a=ω²Asin(ωt) , where A is the amplitude of movement in meters, and ω=2πf, where f is the frequency in Hz. For example, when the frequency is 20 kHz, and the amplitude of vibration 100 µ (100 x 10⁻⁶ m), and maximum acceleration is achieved (i.e., sin(ωt)=1) then acceleration of 1,570,000 m/sec² or about 160,000 g is achieved and can be utilized for delivery. However, it should be appreciated that there exists a reversal proportion between the parameters. For instance, when higher frequency is used, the amplitude can be reduced to achieve similar acceleration rate. At times that ultrasonic transducer is used to create the acceleration stimulus, the high amplitude is essentially created by amplification of the original amplitude of the piezoelectric crystals ,or other source, using a hom or tip preferably designed to be in resonance under operation conditions.

Occasionally, biological component might pass pre-delivery treatment to increase their susceptibility and the efficiency of delivery. Said treatment might for example include adherence of cells which are the target of delivery under *in vitro* conditions, or removal of superficial layers of tissue, such as mucus secretions or keratinized epithelium. According to one non limiting embodiment, the pretreatment might be performed with the same delivery device, operated for instance under streaming or cavitation mode. During such pre-treatment process, a coupling medium shall essentially be present between the accelerating element and the biological component. At times, pre-treatment might be carried out also having gas medium between the driving element and the biological component, and acoustic pressure can be performed to achieve desired pre-treatment effect. Pre treatment, however, might be carried out also using other methods and devices, not part of the current invention.

At times, biological components might pass post-delivery treatment. Said post-delivery treatment might be carried out using the same delivery-device, or methods and devices not part of the current invention or their combination. According to non limiting example, post-delivery treatment might include activation when the delivered agents are irradiation-activated substances. According to one embodiment, the agents might be activated by ultrasound, for instance sonosensitizers such as dimethylformamide, N-methylformamide, or dimethylsulfoxide, or activated by light or other energy modalities after delivery. Substances might be also active in nature, for instance radioactive agents, or activated before, or during their delivery due to ultrasound, light irradiation or other stimuli. According to this example, activated substances are being delivered and effect is performed already during their penetration route so that essentially all the region from the site of administration to the region where the substances reached is essentially destroyed.

According to yet another non-limiting example, post treatment might include controlled degeneration of at least portion of biological component. According to one embodiment, said degeneration is performed after delivery of substances such as vaccines, so creating a biological reservoir for the slow release of substance during normal process of phagocytosis and absorbance of the degenerated tissue. The degeneration might for instance be performed by allowing the accelerating device to touch the biological component for a short period of time, causing friction and degeneration.

In the following the invention will be further illustrated with reference to some non-limiting drawings and examples.

### BRIEF DESCRIPTION OF THE DRAWINGS

**Fig. 1** shows a schematic drawing of the ultrasound delivery device of the invention: **1A** before operation and **1B** during delivery process.
**Fig. 2** shows a schematic representation of a multi-lobed delivery device used in the system of the invention: **2A** before operation and **2B** during delivery process.
**Fig. 3** shows another schematic drawing of a multi lobed device having another substance-supply unit: **3A** before operation and **3B** during supply of substance to be delivered.
**Fig. 4** shows another schematic drawing of delivery device having another actuating mechanism.
**Fig. 5** shows a schematic drawing of a laparoscope-implanted delivery device.
**Fig. 6** shows another schematic drawing of a laparoscope-implanted delivery device, having particular substance supply method and component.
**Fig. 7** shows a schematic drawing of delivery device, having another substance supply method and a concentration element.
**Fig. 8** shows another schematic drawing of a delivery device, having another substance supply method and a dispersion element.
**Fig. 9** shows yet another schematic drawing of a lateral delivery device.

### DETAILED DESCRIPTION OF THE INVENTION

In the device for substances delivery to biological components, high accelerartion rates are utilized to enforce substances delivery from accelerating element to or through biological component, while isolating the biological component from the driving force. The energy is essentially utilized to push the substances, and it essentialy does not reach and consequently is not absorbed in the biological component. The resultant delivery is therefore free of side effects related to energy absorbance and can be performed in superficial as well as in deeper parts of biological components.

A delivery device, in accordance with the invention, functions to deliver solutions as well as particles, yet essentially does not permit the energy to be delivered, therefore prevent the biological component to be affected by the delivery force. As will later be explained, this result is accomplished by drawing continuous vibration and heat away from the biological component.

The delivery system of the invention generally comprises a control unit, a single or a multi-frequency signal generator, a signal amplifier, a matching unit and at least one transducer which may be attached to an amplitude increasing devices, such as resonator or resonating tip. These elements which increases the amplitude, actually increases also the acceleration rate, having small displacement. The phenomena essentially occur at the distal part of said resonating tip, yet might occur also in other locations according to the planning.

At times other means to create high acceleration rate over small displacement might be used either with the ultrasonic driving force or together with other means, or as stand alone means. The high amplitude element is however encased in a housing. The system further comprises substance to be delivered, which may be brought manually or automatically to the accelerating edge by supply elements. The system is provided by a spacer enabling keeping the biological component at certain distance from the accelerating element during operation. This distance might be changed, increased or decreased. At times, for instance during post treatment procedure, distance between resonating element and biological component might be reduced to zero. The system is most preferably also provided with vacuum element, or gas delivery system, to apply low viscosity medium between the accelerating element and the biological component. The vacuum device might be used also for the suction of the non delivered substances from site of delivery, for instance at the end of treatment. The system might be provided also, by element for supply of media for pre-treatment, means for activation of delivered substances, or other means as the case might be.

It is important to note, however, that the system can be operated as stand alone device, for instance during external procedures; It can be further operated as an add-on to other devices, for instance by implanting a high-rate accelerating device, such as resonating transducer, at the distal part of plunger of a gas-spring needle less injection device, or other delivery device, thereby enabling higher acceleration rates to the substances; It can be further operated in laparoscope devices, for delivery to internal biological components, in conjunction with diagnostic element for monitoring the delivery-device location.

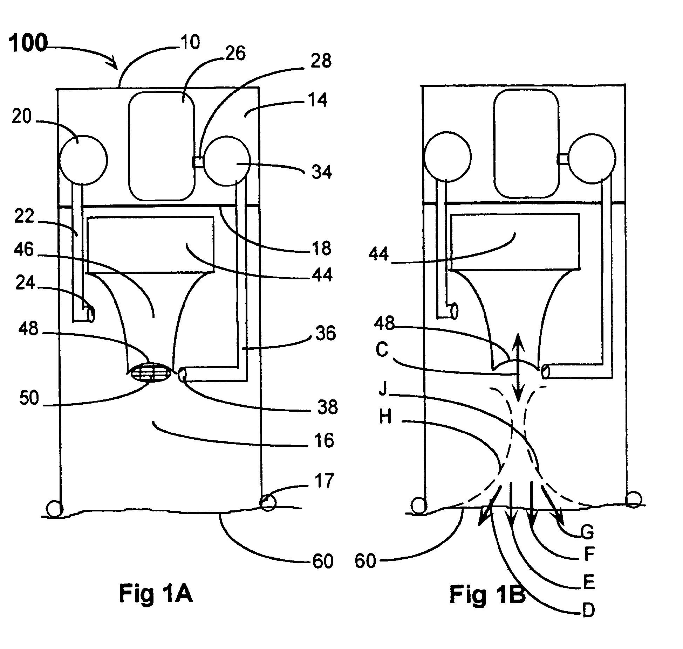

A conceptual model of the delivery device in accordance with the invention, is shown in Figs. **1A** and **1B**. The system operated by electricity, is composed of control unit, a signal generator, a signal amplifier, and matching unit (not shown) which are connected to the said delivery device. The device **100** is encased in housing **10.** It contain a piezoelectric element **44,** transforming the electrical signal to mechanical displacement, and a tip **46** enabling high amplitude at distal end **48,** with still the original high repetition rate. The device contain substance reservoir **26,** linked by tube 28 to pump **34** which delivers said substance via tube **36** and opening **38** to distal end **48.** Element **50** represents the substance accumulated at distal end **48** of the tip **46.** The device is separated to two compartments, by septum **18.** Vacuum pump **20** is having suction activity of air and debris via opening **24** and tube **22.** At the end of the suction activity, the air pressure at space **16** of the septum is lowered. Leakage of gasses from the surroundings is prevented by attaching housing **10** having suction rubber **17** in a shape of circular rim at it's distal open end, to surface **60** of the biological component. The air pressure at space **14** at the other side of septum **18** can remain without change.

In practice, as can be shown in fig **1B**, during activation piezoelectric element **44** transform the electrical signal to mechanical displacement. Maximal amplitude of displacement is achieved at the distal end **48** of the tip, which is displaced and accelerated in the general direction of arrow C. The rapid displacement enforces the substance previously attached to end **48** to be detached and move forward. The acceleration and accompanied forces push the substance in the general direction of arrows **D,E,F** and **G** between schematic broken lines **H** and **J,** into and through surface **60** of the biological component.

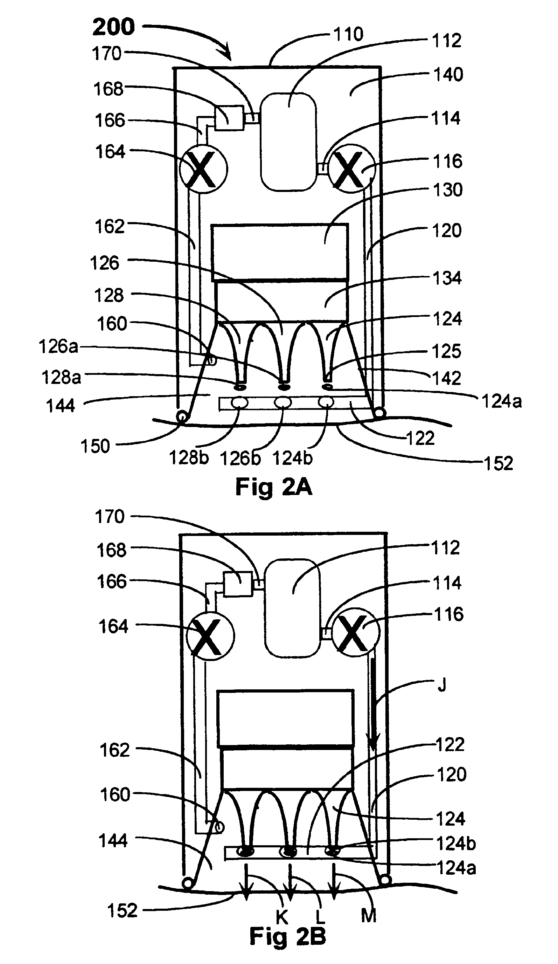

According to the example given is figs **2A** and **2B** a multi lobed device **200** might be used. The device in housing **110** attached to surface **152** of biological component via suction rubber **150,** is composed of a piezoelectric element **130,** guiding hom **134** and distal end composed of several tips **124, 126** and **128.** Attached to, or alternatively part of, the distal ends of the tips, for instance end **125** of tip **124,** are small units **124a, 126a** and **128a ,** capable of absorbing liquids (for instance firm sponge). Substance is supplied from reservoir **112,** to tube **114** and pump **116,** via tube **120** in space **140,** and further to tube **122** in space **144.** Tube **122** has holes **124b, 126b** and **128b,** in the same number of the tips, and in a location compatible to said tips **124, 126** and **128,** respectively. Occasionally, different substances might be delivered from different reservoirs to different tips.

When pump **116** is activated, holes **124b, 126b** and **128b** become filled with substance to be delivered. Suction activity performed by vacuum pump **164,** via opening **160** and tube **162,** reduces gas content in compartment **144.** Suction activity might also facilitate the supply of substance from reservoir **112** to the general direction of space **144,** separated from space **140** by septum **142.** The supply is in the general direction of arrow **J** in Fig **2B****.** It shall be noted that excess of substance in space **144** is carried out via the suction activity into opening **160,** tube **162,** and via pump **164** to tube **166,** filter **168** and back to the reservoir via tube **170.** As demonstrated in Fig **2B****,** during operation distance between tips, for instance tip **124** and its absorbing unit **124a** on one hand, and holes, for instance **124b** on the other hand, is diminished. Supplied substance enters absorbing unit **124a**, and similarly enters **126a** and **128a**, and the accelerating tip **124,** and similarly **126** and **128,** deliver substance towards surface **152** in the general direction of schematic arrows **K,L** and **M.**

It shall be appreciated that as pre-treatment, space **144** might be filled with gassed distilled water, and cavitation performed using irradiation via tips **124,126** and **128.** At the end of said pre-treatment water shall be pumped out via suction hole **160.**

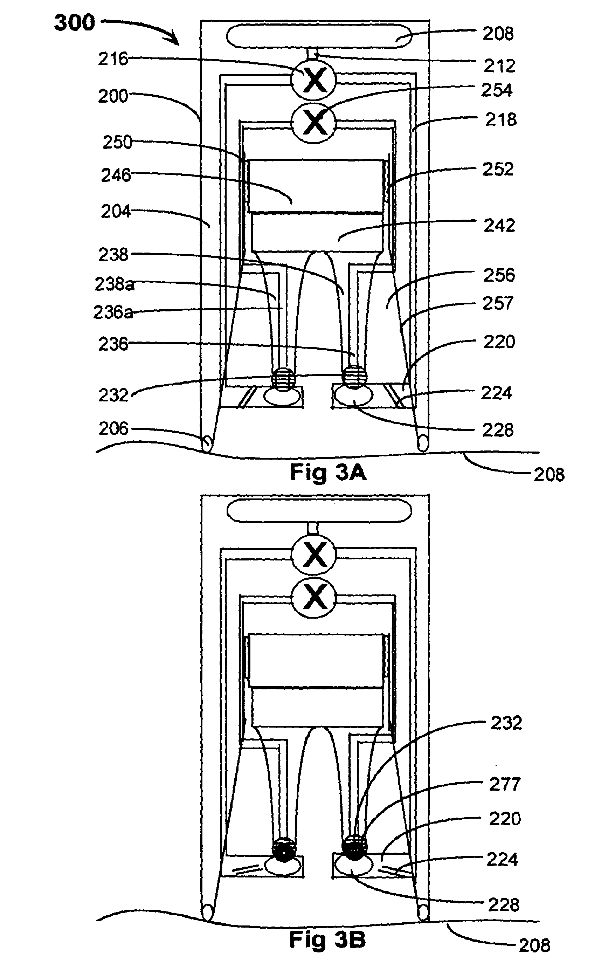

Fig **3** schematically describes device **300** of the invention. Delivery device is encased in housing **200** attached to the biological component via rubber ring **206.** Piezoelectric transducer **246** is coupled to tips **238** and **238a** via coupling hom **242.** The transducer is attached to inner wall **257,** separating between spaces **204** and **256,** via attachment unit **252** that also prevents leakage of gasses between spaces. Substance, for instance in the form of particles, is kept in reservoir **208,** from where it can be delivered via tube **212** and pump **216,** to tube **218** and tube compartment **220.** Said tube compartment **220** has hollowed area **228** and a valve **224.** At the distal end of each tip **238** and **238a,** a lattice **232** is present. The tips are partially hollowed; From lattice **232** tube **236** and **236a** run, via tube **250** into vacuum pump **254.** During supply of substance, or at certain synchronization with supply of substance, suction activity of pump **254,** opens valve **224** to allow particles to be supplied to hollowed area **228.** Substances then are accumulated at lattice **232** and form aggregate **277.** Ultrasonic pulse will deliver particles of aggregate **277** towards and into surface **208** of biological component.

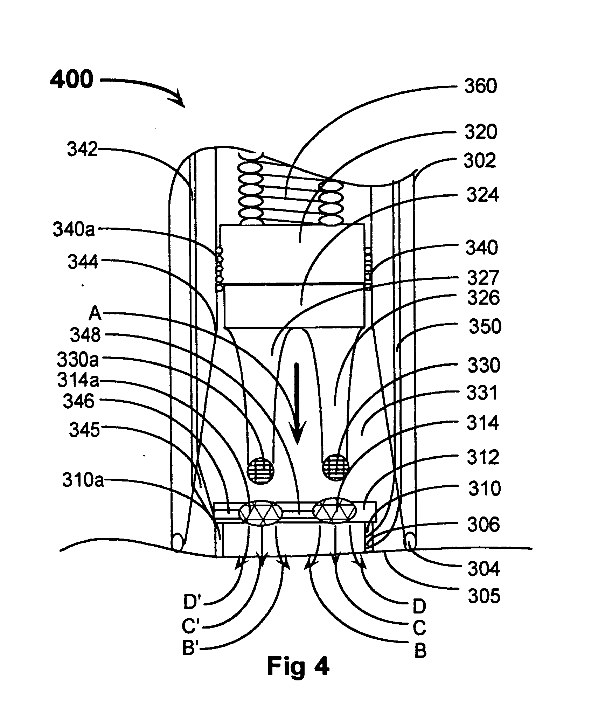

System **400** of Fig. **4** describes another non limiting example of a device. According to this embodiment, inside housing **302** attached to biological component **305** via rubber ring **304,** the accelerating element is attached at its proximal end to spring **360.** The accelerating element, composed of proximal ultrasonic transducer **320,** guiding horn **324** and tips **326** and **327,** is attached to inner wall **344** via rings **340** and **340a**, that serve also to prevent gas transfer between compartments. However, operation might be performed also when similar pressure exists in the different compartments. Certain degree of vacuum of space **331** is carried out by suction activity of vacuum pump (not shown), via tube **350** and opening **306.** Supply of substance is carried out from reservoir and pump (not shown) via tube **342,** passing wall **344** via tube **345,** via tube **346** into lattice or absorbent element **314a** (for particles or solutions respectively) and further via tube **348** to lattice or absorbent **314.** Both **314** and **314a,** and the interconnecting tubes, are located on stab **312** kept at certain distance from surface of biological component **305,** by legs **310** and **310a**.

During actuation, releasing of spring **360,** causes movement of the accelerating element in the general direction of arrow **A**, towards lattice/absorbent **314** and **314a.** When distal parts **330** and **330a** having high accelerating rate, of accelerating element, touches area **314** and **314a,** substance located in said **314** and **314a** is accelerated and delivered towards surface **305** of biological component in the general direction of schematic arrows **B,C,D,B', C'** and **D'.** The whole inner construction might be also circular, for instance circular shape of tip, circular shape of lattice or absorbent and so on. The impact of contact between vibrating edges **330** and **330a,** and elements **314** and **314a** on the other hand, causes the accelerating element to move backwards with spring **360,** new substance is applied to **314** and **314a**, and the procedure is being repeated. At the end of procedure, as post-treatment, edges **330** and **330a** can be vibrated while attached to surface **305,** thereby causing local destruction at surface **305.** It will be followed by slow release of substances, where the biological component itself serves as reservoir. Post treatment might also for instance include activation of sonosensitizers, previously delivered to biological component.

It shall be appreciated, that with few modifications, the schematic device described in Fig **4** might be also utilized as add-on that significantly improves performance of, for instance gas spring actuated injection devices, of for instance Medi-Ject Cooperation, or Bioject, Inc., Genesis Medical Technologies, Inc., Weston Medical LimitedRymed Technologies, Mycone Dental Supply Co. Ferton Holding and the like. According to a non-limiting embodiment part of the present invention, at the front edge of piston, or gas releasing orifice, or elsewhere, a high accelerating agent such as ultrasonic element of high frequency and relatively high amplitude (preferably tenth of millimeter), or edge of ultrasonic vibrating tip, is placed to further accelerate substances, in addition to the spring or gas pressure originally used.

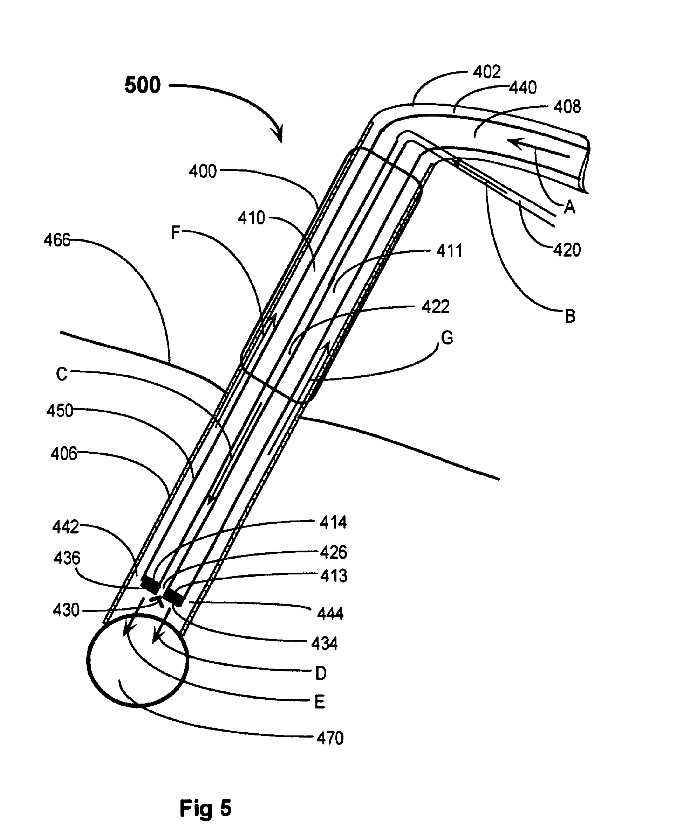

Fig **5** schematically describes device **500** for delivery to internal tissues. The system is composed of control unit, signal generator, amplifier, matching unit and transducer, as well as possibly increasing amplitude element such as tip, all of which are not shown. Movements created by the transducer (not shown) are transferred to the treatment device, via a wave guide **408** in the general direction described by arrow A. The wave guide is designed so its dimensions till ends **413** and **414** enable movement at resonance of distal ends **413** and **414.** Space **440** between wave-guide and wave-guide sleeve **402** is preferably under certain vacuum conditions, as will be further explained below. Tube **420** enters said wave-guide at a point which is preferably a point of minimal movement, for instance zero point. Tube **420** supply the substances from a reservoir (not shown) in the general direction of arrow **B,** and further via continuation tube **422** in the general direction described by arrow **C**. Tube **422** might be a hollowed area of a rounded wave guide, and then parts **410** and **411** actually refer to two sides of a cylinder, but it can be also a channel between two separated (and for instance flat), wave guides **410** and **411.** Supplied substances leave tube **422** via opening **426,** reaches reflecting valve **430,** and are reflected and accumulated in lattice, or sponge, **434** and **436** (for particles or liquid), which again might be two sides of a cylindrical component.

Certain degree of vacuum is created by a pump (not shown). Suction of air, cellular debris or excess of substance is performed from the area between delivery device **500** and internal biological component **470.** Suction is performed via the spaces **442** and **444,** between device laparoscope-wall **406,** and wall **450** of accelerating element of the wave-guide **410** and **411,** in the general direction of schematic arrows **F** and **G.** The supply of substances might be via pushing them with a pump via tube **420,** but also by suction activity from the reservoir.

During activity, while held by handle **400,** and while wall **406** serves as laparoscope guide, delivery device is inserted via surface **466** to desired location, for instance organ **470.** Certain degree of vacuum, according to the needs is performed so that at least space between elements **436** and **434** on one hand, and organ **470** essentially contains no liquid or cellular debris. Substance is delivered to be accumulated in elements **434** and **436.** Accelerating element is operated to create high amplitude repeated movement of ends **413** and **414** of the wave-guide. Substance accumulated in **436** and **434** is accelerated towards and into organ **470** in the general direction of arrows **D** and **E.** According to non-limiting example, organ 470 might be a tumor and the substance to be delivered composed of Tumor Necrosis Factor.

Generally speaking, the suction activity and the accompanied reduction of air pressure, aim at increasing the isolation capabilities of the space between biological component and accelerating element, and concomitantly to reduce friction of the accelerated substance and air. It shall be noted, however that procedure can be performed also via gasses, and other media.

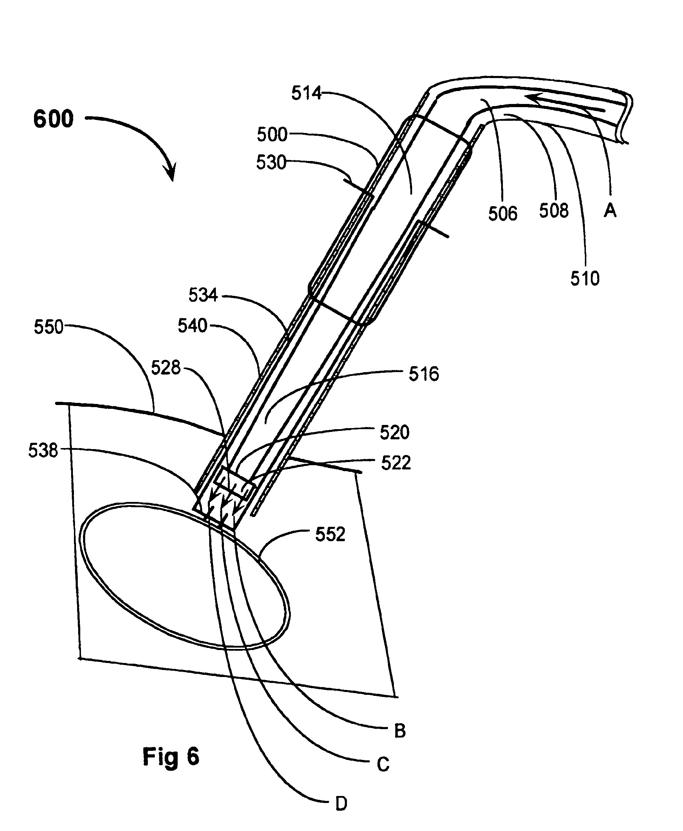

Fig **6** schematically describes device **600** for delivery to internal tissues. The system is composed of control unit, signal generator, amplifier, matching unit and transducer, all of which are not shown. Movements of high accelerating rate, created by the transducer (not shown) are transferred in the general direction of schematic arrow A, via wave-guide **506.** Wave guide **506,** is mechanically isolated from sleeve **510** by space **508,** containing gas or slight degree of vacuum. Similar isolation exists also between the other accelerating components, such as tip **514** or delivery distal end **522,** and laparoscope cover **540.** The tip has larger cross section in area **514,** and lower cross section closer to the distal end, at area **516,** and therefore amplitude of movement is increased under the same frequency and acceleration rate is increased. The whole device is designed for activity under resonance, so that area of maximal movement, and maximal accelerating rate, is at distal end **520** of the tip. The space between tip end and lattice wall **528** contain the substance to be delivered.

At times, a device where the substance fills the wave-guide, might be used. In such case, a liquid substance medium, or gel with appropriate substance to be delivered, is the content of at least last portions of the wave guide, including for instance area **506, 514** and **516** and with continuity to the area between tip end **520** and lattice **528,** for instance via openings in tip end **520.** Alternatively, the wave-guide may be composed of solid material, or liquid not relevant for the delivery, and for instance only the space between **520** and **528** with said substance to be delivered.

During operation, the device held in handle **500 ,** is inserted via surface **550** of biological component till target **552,** having laparoscope wall **540** as guiding element. During insertion, hollowed grid-like end **538** is in same line as end of wall **540.** When laparoscope wall reaches target **552,** insertion stops. At this stage, pushing of sub-handle **530,** transfer further movement of hollowed grid-like end **538,** via walls of cylinder **534.** Movement of grid-like end **538** might press a bit target **552,** but in addition it increases the distance between lattice **528** on one hand, and hollowed grid-like **538** and target **552** on the other hand. This increase of distance is performed and concomitantly, or shortly after and essentially before the increased distance is filled by liquids, waves are emitted and high acceleration is performed to affect tip end **522.** It further accelerates substance via lattice **528** which might have larger area than tip end **522,** and via hollowed grid-like end **538,** into target **552,** in the general direction of arrows **B,C** and **D.** The ultrasonic path might be also constructed in a different way, so having for instance the ultrasonic transducer in handle **500.**

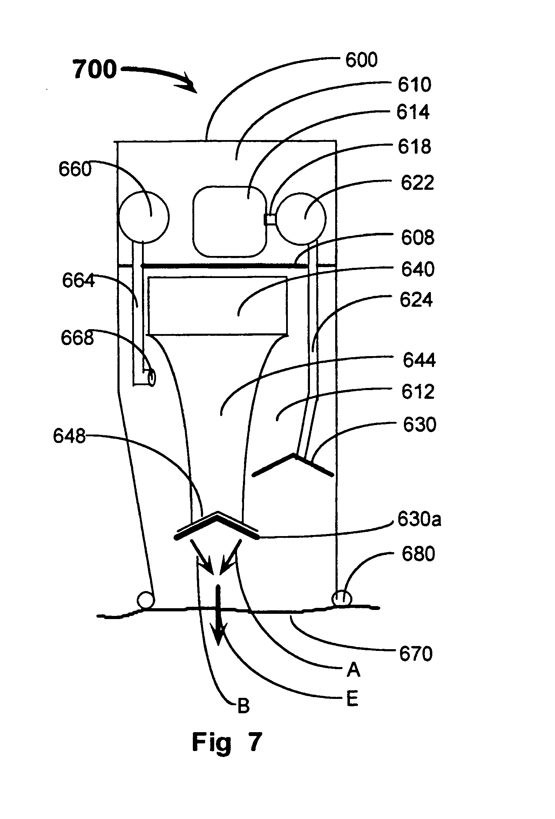

Fig. **7** schematically describes an example of delivery device **700,** encased in housing **600** which is attached to biological component **670** via suction rubber **680.** Control unit, generating and amplifying elements of the system are not shown. Transducer **640,** might be in housing **600,** yet might be also located elsewhere, with a wave-guide for transferring the movements to the treatment device, subject of this schematic drawing. Septum **608** separated the device to normal pressure zone **610** and low pressure zone **612,** whereas low pressure is created via suction activity employed by pump **660** via opening **668** and tube **664.** Delivered substance might be in encapsulated as upside v-shaped **630,** and brought from reservoir **614** via guiding element **614,** and motor **622,** utilizing arm **624.**

During operation, mechanical signal given by the transducer, is amplified in amplitude and acceleration rate in tip **644.** Maximal, or at least optimal, acceleration rate is achieved in upside v-shaped tip end **648.** Substance **630a,** or its components, located attached to tip-end **648,** are accelerated in the general direction vectors schematically described as arrows **A** and **B.** The vectors created, are being further united and amplified in the general direction of schematic delivery vector **E,** through surface **670** into the biological component.

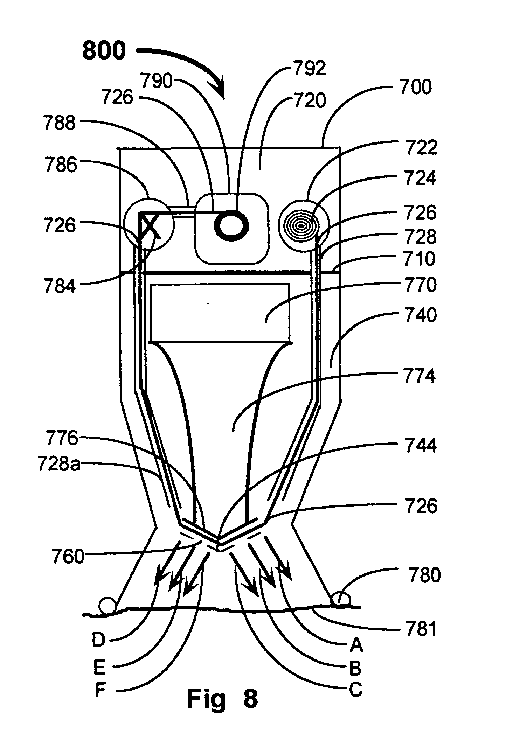

Fig. **8** describes delivery device **800,** encased in housing **700** which is attached to biological component surface **781** via rubber ring **780.** Septum **710,** divides it to space **720** and space **740,** whereas space **740** is preferably having slight vacuum. The accelerating elements of the device is composed of transducer **770,** guiding tip element **774** and distal v-shaped end 776, having the appropriate acceleration rate. Substance is in a strip form. Bulk of substance **724,** is located in sub-encasing **722,** from where strip **726** is supplied via channel **728.** At least one side of strip **726** contains the substance to be delivered. Strip is forwarded via the space between v-shaped distal end **776** and v-shaped lattice **744,** and further via channel **728a.** The supply of the strip is carried out by pulling activity, performed by motor **784** in casing **786.** It pulls the strip from reservoir **724,** as herein above described and further via tube **788** to reservoir **792** of substance depleted strip, in sub-housing **790.**

During operation, strip is moving from reservoir **724** to reservoir **792,** partially along accelerating v-shaped end **776,** and substance is accelerated via openings **760** of lattice **744 ,** in the general direction of arrows **A,B,C,D,E** and **F** towards and into surface **781.** Operation can be performed in continuous mode, for instance continuous movement of strip together with continuous activation of accelerating element. Operation can be done also in synchronized mode, for instance movement of strip, activation of acceleration, cessation of activation, movement of strip and so on. Combined mode might be also performed.

According to a non limiting embodiment, substances attached to strip are inert solid crystals. Their acceleration at certain angle and acceleration rate towards biological component, will cause during impingement energetic impact on surface of biological component and removal of sub components or layers therefrom. Said debris can be further removed, for instance by a suction activity.

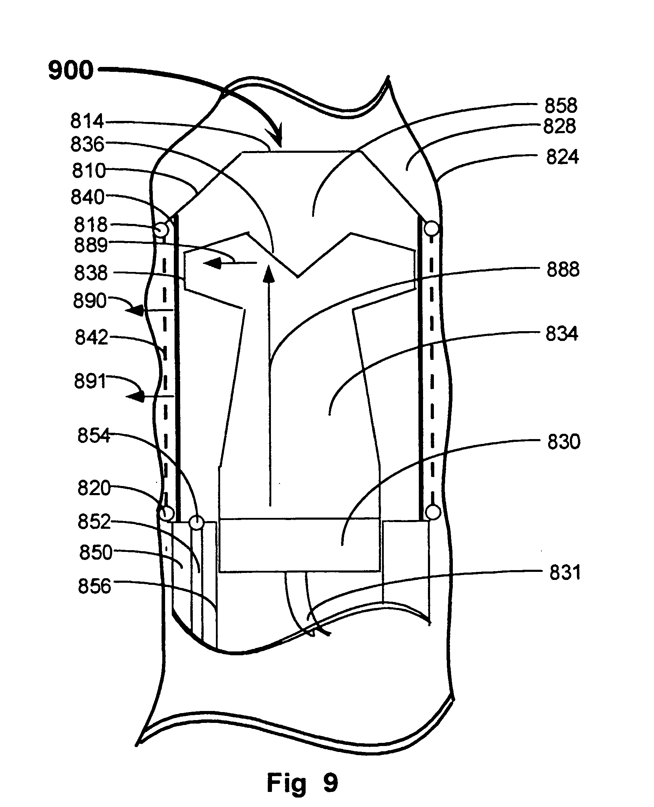

Fig **9** schematically describes lateral delivery device **900,** the delivery component of delivery system. The device might be cylindrical, encased in cylindrical housing **810** having narrow leading edge **814.** The device **900** according to this non-limiting example is located in lumen **828** of tube-like biological component **824** which might be for instance be vagina or the coronary blood vessels. Leading edge **814,** which essentially is narrow then at least part of other components of the device, widened biological component while being inserted to it, and the biological component is then supported and clasped on the area between rings **818** and **820.**

The accelerating element is transducer **830,** receiving the electrical signal via cable **831** to create transmission of waves and acceleration of movement. Acceleration of movement is increased via wave guide tip **834.** The general direction of propagation of stimuli is from the transducer **830,** via wave guide tip in the general direction of schematic arrow **888,** reflected from wall **836** in the general direction of schematic arrow **889** and till edge **838** having maximal amplitude and maximal acceleration rate. The surface of the device between rings **818** and **820** is composed of cylindrical lattice cover **842,** and inner to it cylindrical reservoir sheet **840** that contain the substance to be delivered. Said substance might for instance be vaccine for local immunization or the vaginal epithelium, localized immune suppression before introducing an IUD, or substance for after-widening stabilization of the coronary arteries, similar to stents, or compounds for paving the coronary arteries before implantation of stents.

After the device reaches its place, certain reduction of the atmospheric pressure in space **858** is created, by suction activity via opening **854** of suction tube **852** of guiding element **850.** Stimuli is then created in the transducer, waves are emitted so that edge **838** is accelerated. The acceleration causes delivery of substance from reservoir sheet **840** via opening of lattice **842** and into biological component **824** in the general direction of schematic arrows **890** and **891.** The device might be operated also without lattice **842,** providing that a certain space can be kept between reservoir **840** and biological component **824.** Said space shall preferably be composed of low density medium.

At times, the delivery device might be operated in such synchronization that substances delivered in a circular way, for instance in direction of arrow **890,** will get harder after delivery for the creation of a solid ring for mechanical support. That way several rings, with possible supportive linking elements, or any other shape performed according to the lattice design and construction, might be created for establishing for instance a new type of in-situ constructed stent for the stabilization of coronary blood vessels, urethra and other vessels.

During operation, or in synchronic manner, the accelerating device is pulled backwards where transducer **830** is guided along inner wall **856** of guiding element **850.** That way each time is affects and delivers substance from another area of reservoir sheet **840.** According to non limiting embodiment, the transducer is located outside the delivery device, closer to the other system component such as signal generator, control panel or suction pump, and only appropriate wave guide is located in the device to create the delivery.

It shall be appreciated that also here same device can be used initially to remove portion of tissue, suction for removal of debris, and subsequently the delivery of for instance substances for mechanical support such as for coronary stent or for immunization and the like.. The control unit can for example monitor and determine gas pressure in the delivery device, amplitude of vibration, frequency, pulse duration, duty cycle of emitted waves, movement of accelerating element in relation to the biological component or to the supplied substance, rate of supplying the substance and other parameters that might be relevant.

The description and drawings were given for illustrative and non limiting purposes only. The invention is defined by the claims.

## Claims

1. A device (100) for the administration by acceleration of substances to biological components, comprising means capable of producing a driving force in the form of a cyclic accelerating movement over a displacement of amplitude, enabling acceleration of a substance *per-se* to be delivered, rapid displacement of the substance and driving the substance to penetrate a surface (60) of the biological component, while isolating the bulk of the targeted biological component from the driving force during delivery, **characterised in that** said means comprises:
a transducer (44) having a transducer tip (46) configured to amplify at a distal end thereof (48), displacement at a proximal end of said transducer tip produced by the transducer (44); and
a compartment (16) configured to enclose a vacuum or a gaseous medium and configured to isolate the targeted biological component from the driving source, said compartment having an end adapted to be placed in contact with the biological components, wherein dimensions of said compartment are sufficient to permit the substance to accelerate therethrough.

2. A device according to claim 1, wherein the driving force is emitted by ultrasound member provided in the form of an ultrasonic transducer capable of producing ultrasonic vibration and delivery is performed by accelerating the substance with an ultrasonic vibrating element.

3. A device according to claim 1, **characterized in that** the accelerating movement is emitted for a time period equivalent to at least a quarter of a cycle and at least one stimulus is being given, wherein the substance to be administered is coupled to the stimulating element during at least part of the stimulus period and essentially till desired delivery is carried out.

4. A device according to claim 2, wherein the transducer (44) is operable at a frequency of 1 Hz to 30 MHz, displacement produced at the distal end (48) of the tip (46) has an amplitude of 1 micron to 10,000 microns, and said transducer tip (46) is capable of being operated to produce power density of between of 0.0001 W/cm² to 10,000 W/cm².

5. A device of claim 1, further comprising a substance delivery pump adapted to deliver substance that might be liquid, gel, paste, powder, particles, pellet, solid strip, sleeve and the like.

6. A device according to claim 2, having a treatment mode and pre-treatment or a post-treatment mode wherein the device can further perform steps of pre-administration tissue treatment, or post-administration tissue treatment, such as tissue degeneration by attaching the vibrating element used for the acceleration, to the tissue.

7. A device according to claim 1, for the administration by acceleration of substances to biological components comprising:
at least one acceleration agent capable of giving acceleration stimulus to the substance per-se, and having acceleration surface that can be of different shapes,
at least a portion of substance to be delivered,
means for supply of substance to be delivered to site of acceleration.

8. The device of claim 7, wherein during delivery a space medium exists between substance to be delivered by acceleration and biological component.

9. The device of claim 1 further including a vacuum pump (20) for producing vacuum or negative pressure in said isolating means.

## Patentansprüche

1. Vorrichtung (100) zur beschleunigten Verabreichung von Substanzen an biologische Komponenten, welche ein Element enthält, welches zur Erzeugung einer Antriebskraft in der Form von einer zyklischen Beschleunigungsbewegung über einen Amplitudenversatz fähig ist, welches eine beschleunigte Zuführung von einer Substanz an sich, eine schnelle Absetzung der Substanz und einen Antrieb der Substanz zum Durchdringen von einer Oberfläche (60) von der biologischen Komponente während einer Isolation des Massenanteils der gezielten biologischen Komponente von der Antriebskraft während der Zuführung ermöglicht, **dadurch gekennzeichnet, dass** das Element enthält:
einen Übertrager (44), welcher eine Übertragerspitze (46) hat, welche dazu ausgelegt ist, an einem distalen Ende davon (48) eine Absetzung an einem proximalen Ende von der Übertragerspitze, welche durch den Übertrager (44) erzeugt ist, zu verstärken; und
eine Kammer (16), welche dazu ausgelegt ist, ein Vakuum oder ein gasförmiges Medium zu umgeben, und dazu ausgelegt ist, die gezielte biologische Komponente von der Antriebskraft zu isolieren, wobei die Kammer ein Ende hat, welches dazu ausgelegt ist, mit den biologischen Komponenten in Kontakt gesetzt zu werden, wobei Ausmaße von der Kammer dazu ausreichen, eine Beschleunigung von der Substanz dort hindurch zu ermöglichen.

2. Vorrichtung nach Anspruch 1, bei welcher die Antriebskraft durch ein Ultraschallelement emittiert wird, welches in der Form von einem Ultraschall-Übertrager bereitgestellt ist, welcher dazu in der Lage ist, eine Ultraschall-Vibration zu erzeugen, und wobei die Zufuhr durch ein Beschleunigen von der Substanz mit einem Ultraschall-Vibrationselement durchgeführt ist.

3. Vorrichtung nach Anspruch 1, **dadurch gekennzeichnet, dass** die Beschleunigungsbewegung innerhalb von einer Zeitperiode emittiert ist, welche gleich zumindest ein Viertel eines Zyklus ist, und wobei zumindest eine Anregung abgegeben ist, wobei die zu verabreichende Substanz innerhalb von zumindest einem Teil von der Anregungsperiode und im Wesentlichen, bis die gewünschte Verabreichung durchgeführt ist, mit dem Anregungselement gekoppelt ist.

4. Vorrichtung nach Anspruch 2, bei welcher der Übertrager (44) bei einer Frequenz von 1 Hz bis 30 MHz betriebsbereit ist, wobei eine Absetzung, welche am distalen Ende (48) von der Spitze (46) erzeugt ist, eine Amplitude von 1 Mikron bis 10.000 Mikron hat, und wobei die Übertragerspitze (46) dazu in der Lage ist, dazu betrieben zu werden, eine Leistungsdichte zwischen 0,0001 W/cm² und 10.000 W/cm² zu erzeugen.

5. Vorrichtung nach Anspruch 1, welche ferner eine Substanz-Zufuhrpumpe enthält, welche dazu ausgelegt ist, eine Substanz zuzuführen, welche eine Flüssigkeit, ein Gel, eine Paste, ein Pulver, Partikel, Pellets, feste Streifen, Hülsen und dergleichen sein kann.

6. Vorrichtung nach Anspruch 2, welche einen Behandlungs-Modus und einen Vorbehandlungs- oder Nachbehandlungs-Modus hat, wobei die Vorrichtung ferner Schritte zur Vorverabreichung einer Gewebebehandlung oder Nachverabreichung einer Gewebebehandlung durchführen kann, wie beispielsweise eine Gewebe-Degeneration, durch Anbringen des Vibrationselements, welches zur Beschleunigung verwendet wird, an das Gewebe.

7. Vorrichtung nach Anspruch 1 zur beschleunigten Verabreichung von Substanzen an biologische Komponenten, welche enthält:
zumindest ein Beschleunigungsmittel, welches zum Anlegen von einer Beschleunigungs-Anregung an die Substanz an sich in der Lage ist, und eine Beschleunigungsoberfläche hat, welche unterschiedliche Formen haben kann, zumindest einen Anteil einer zuzuführenden Substanz, ein Element zur Zufuhr einer zuzuführenden Substanz an eine Beschleunigungsseite.

8. Vorrichtung nach Anspruch 7, bei welcher innerhalb der Zufuhr ein Raummedium zwischen einer durch Beschleunigung zuzuführenden Substanz und einer biologischen Komponente vorliegt.

9. Vorrichtung nach Anspruch 1, welche ferner eine Vakuumpumpe (20) zum Erzeugen eines Vakuums oder eines Unterdrucks in dem Isolationselement enthält.

## Revendications

1. Dispositif (100) pour l'administration par accélération de substances à des composants biologiques, comprenant des moyens capables de produire une force d'entraînement sous la forme d'un mouvement d'accélération cyclique sur un déplacement d'amplitude, de permettre une accélération d'une substance *per-se* à administrer, un déplacement rapide de la substance et d'amener la substance à pénétrer une surface (60) du composant biologique, tout en isolant la majeure partie du composant biologique visé de la force d'entraînement pendant l'administration, **caractérisé en ce que** lesdits moyens comprennent :
un transducteur (44) possédant une pointe de transducteur (46) configurée pour amplifier, au niveau d'une extrémité distale de celui-ci (48), un déplacement produit par le transducteur (44) au niveau d'une extrémité proximale de ladite pointe de transducteur ; et
un compartiment (16) configuré pour renfermer un vide ou un milieu gazeux et configuré pour isoler le composant biologique visé de la source d'entraînement, ledit compartiment possédant une extrémité adaptée pour être placée en contact avec le composant biologique, les dimensions dudit compartiment étant suffisantes pour permettre à la substance d'accélérer à travers celui-ci.

2. Dispositif selon la revendication 1, dans lequel la force d'entraînement est émise par un élément ultrasonique fourni sous la forme d'un transducteur ultrasonique capable de produire une vibration ultrasonique, et l'administration est réalisée en accélérant la substance avec un élément vibrant ultrasonique.

3. Dispositif selon la revendication 1, **caractérisé en ce que** le mouvement d'accélération est émis pour une période de temps équivalente à au moins un quart d'un cycle et au moins un stimulus est fourni, la substance à administrer étant couplée à l'élément de stimulation pendant au moins une partie de la période de stimulus, essentiellement jusqu'à ce que l'administration souhaitée ait été mise en oeuvre.

4. Dispositif selon la revendication 2, dans lequel le transducteur (44) peut fonctionner à une fréquence de 1 Hz à 30 MHz, le déplacement produit au niveau de l'extrémité distale (48) de la pointe (46) a une amplitude de 1 micron à 10 000 microns, et ladite pointe de transducteur (46) est capable de fonctionner pour produire une densité de puissance comprise entre 0,0001 W/cm² et 10 000 W/cm².

5. Dispositif selon la revendication 1, comprenant en outre une pompe d'administration de substance adaptée pour administrer une substance qui peut être un liquide, un gel, une pâte, une poudre, des particules, des pastilles, une bande solide, une enveloppe et similaire.

6. Dispositif selon la revendication 2, possédant un mode de traitement et un mode de prétraitement ou un mode de post-traitement, dans lequel le dispositif peut en outre réaliser des étapes de traitement de tissu préalable à l'administration, ou de traitement de tissu postérieur à l'administration, comme une dégénérescence de tissu, en attachant au tissu l'élément vibrant utilisé pour l'accélération.

7. Dispositif selon la revendication 1, pour l'administration par accélération de substances à des composants biologiques, comprenant :
au moins un agent d'accélération capable de donner un stimulus d'accélération à la substance *per-se,* et possédant une surface d'accélération qui peut être de différentes formes,
au moins une quantité de substance à administrer,
des moyens de fourniture de la substance à administrer vers le site d'accélération.

8. Dispositif selon la revendication 7, dans lequel, pendant l'administration, un milieu spatial existe entre la substance à administrer par accélération et le composant biologique.

9. Dispositif selon la revendication 1, comprenant en outre une pompe à vide (20) pour produire un vide ou une pression négative dans lesdits moyens d'isolation.
